# EUROPÄISCHE PATENTSCHRIFT

(11) **EP 0 728 731 B1**
(45) Veröffentlichungstag und Bekanntmachung des Hinweises auf die Patenterteilung: **01.09.1999**
(21) Anmeldenummer: 96101885.0
(22) Anmeldetag: 09.02.1996
(51) Int. Cl.: C07C 67/303, C07C 69/40

(54) **Verfahren zur Herstellung von Bernsteinsäure-dialkylestern**
Process for the preparation of dialkyl esters of succinic acid
Procédé de production d'esters dialkyliques d'acide succinique

(30) Priorität: 22.02.1995 DE 19506096
(43) Veröffentlichungstag der Anmeldung: 28.08.1996
(73) Patentinhaber: BAYER AG, 51368 Leverkusen (DE)
(72) Erfinder: Darsow, Gerhard, Dr., D-47804 Krefeld (DE)

(56) Entgegenhaltungen:
- EP-A- 0 008 727
- US-A- 3 830 830

## Beschreibung

Die vorliegende Erfindung betrifft ein kostengünstiges, kontinuierlich arbeitendes Verfahren zur Herstellung von Bernsteinsäure-dialkylestern aus Maleinsäure-dialkylestern, bei welchem nur sehr geringe Mengen des üblicherweise bei der Hydrierung von Maleinsäureestern entstehenden Butandiols-1,4 sowie keine Mono-und keine Hydroxycarbonsäuren der C-Zahlen <4 gebildet werden.

Bernsteinsäure-dialkylester sind wichtige, biologisch gut abbaubare Lösungsmittel für Lacke und Weichmacher für thermoplastische Polyester mit besonderen mechanischen und chemischen Eigenschaften.

Es ist bekannt, Bernsteinsäure-dialkylester durch Veresterung von Bernsteinsäure oder Bernsteinsäureanhydrid mit den entsprechenden Monoalkoholen herzustellen, wobei häufig acide Komponenten als Veresterungskatalysatoren zum Einsatz kommen und mit hohen Alkoholüberschüssen gearbeitet wird. Es ist ferner bekannt, Maleinsäure-dialkylester mit Wasserstoff in einem Suspensionsverfahren mit einem pulverförmigen Pd/Al₂O₃-Katalysator diskontinuierlich zu den korrespondierenden Bernsteinsäure-dialkylestern zu hydrieren (EP 190 424).

Der Reaktionsverlauf läßt sich durch das folgende Reaktionsschema veranschaulichen:

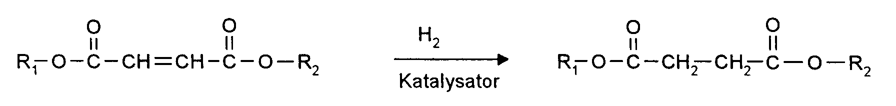

Hierbei können R₁ und R₂ gleiche oder unterschiedliche n- oder iso-Alkylreste der C-Zahlen 1 bis 12 oder cyclische Alkylreste der C-Zahlen 3 bis 6 sein.

Diskontinuierliche Verfahren haben den Nachteil, daß ihre Kapazität relativ zum Reaktionsvolumen sehr klein ist und somit ein Bedarf nach großen Reaktionsapparaturen und Lagertanks besteht. Energieverbrauch und Personalbedarf sind verhältnismäßig hoch.

Kontinuierliche Pulverkatalysatorverfahren, die mit mehreren in Kaskade geschalteten Hydrierreaktoren arbeiten, vermeiden einen Teil dieser Nachteile. Es bleibt jedoch das Erfordernis, die pulverförmigen Katalysatoren mehrfach gezielt zu dosieren, umzupumpen und quantitativ vom Reaktionsprodukt abzufiltrieren. Die Katalysatorschlammpumpen unterliegen einem hohen mechanischen Verschleiß. Die quantitative Entfernung der pulverförmigen Katalysatoren aus dem Reaktionsprodukt ist aufwendig. Ferner ist die Gefahr groß, die Katalysatoraktivität durch die zusätzlichen Operationen verhältnismäßig schnell zu verringern. Es ist daher vorteilhaft, die Reaktion über fest angeordneten Katalysatoren ablaufen zu lassen. Solche Katalysatoren müssen eine hohe Aktivität besitzen, die über einen längeren Zeitraum nicht nachlassen darf, weil häufige Katalysatorwechsel bei Festbettreaktionen ebenfalls aufwendig sind.

Überraschenderweise wurde nun gefunden, daß man Maleinsäure-dialkylester in hohen Ausbeuten kontinuierlich über im Festbett angeordneten trägerfreien Formkörpern aus sauerstofffreien Metallpulvern von einem oder mehreren Elementen der Eisenuntergruppe der VIII. Nebengruppe des Periodensystems (Mendelejew) zu den korrespondierenden Bernsteinsäure-dialkylestern hydrieren kann. Dazu kann es nützlich sein, die Metalle der Eisenuntergruppe mit aktivierend wirkenden Elementen der VI. Nebengruppe des Periodensystems zu legieren. Dabei können die zum Einsatz kommenden Pulver zusätzlich kleine Anteile nicht katalytisch wirkender Elemente (z.B. Silizium, Aluminium, Titan, Kohlenstoff) enthalten, ohne daß die hohe Aktivität gemindert wird. Die Festkörper müssen eine Druckfestigkeit von 20 - 250 N und eine innere Oberfläche von 10 -80 m²/g aufweisen.

Zum Einsatz kommen vorzugsweise Maleinsäure-dialkylester mit einer Reinheit ≥99 %. Es lassen sich aber auch Maleinsäure-dialkylester mit geringerer Reinheit nahezu quantitativ umsetzen.

Gegenstand der Erfindung ist damit ein Verfahren zur kontinuierlichen Herstellung von Bernsteinsäure-dialkylestern durch katalytische Hydrierung von Maleinsäuredialkylestern, das dadurch gekennzeichnet ist, daß die Hydrierung in der flüssigen Phase bei einem H₂-Druck von 50 bis 400 bar, einer 20 bis 60-fachen molaren H₂-Menge, bezogen auf die stöchiometrische Menge, und bei einer Temperatur von 30 bis 160°C an im Festbett angeordneten sauerstofffreien und trägerfreien Katalysatoren durchgeführt wird, die als verpreßte aus Metallpulvern hergestellte Formkörper vorliegen, die eine Druckfestigkeit von 20 bis 250 N und eine innere Oberfläche von 10 bis 80 m²/g aufweisen und bei denen die Metallpulver 60 bis 100 Gew.-% eines oder mehrerer Eisenmetalle, 0 bis 15 Gew.-% eines oder mehrerer Metalle der VI. Nebengruppe und 0 bis 25 Gew.-% eines oder mehrerer hydrierinerter Elemente aus der Gruppe von Aluminium, Silicium, Titan und Kohlenstoff, alles bezogen auf das Gesamtgewicht des Metallpulvers, enthalten.

Die Druckfesfigkeit der trägerfreien Formkörper kann nach DIN 50 106 bestimmt werden.

Die Überprüfung von trägerfreien Formkörpern auf die anspruchsgemäßen inneren Oberflächen und damit auf Brauchbarkeit für das erfindungsgemäße Verfahren kann nach Methoden durchgeführt werden, die von F.M. Nelsen und F.T. Eggertsen, Analyt. Chem. 30 (1958), S. 1387-1390 bzw. S.J. Gregg und K.S.W. Sing, Adsorption, Surface Area and Porosity, London 1982, Kap. 2 und 6, beschrieben worden sind.

Die Eisenuntergruppe der VIII. Nebengruppe des Periodensystems (Mendelejew) enthält die Elemente Eisen, Kobalt und Nickel. Die erfindungsgemäß zu verwendenen trägerfreien Formkörper enthalten eines oder mehrere dieser Metalle in Mengen von mindestens 60, vorzugsweise mindestens 70, insbesondere mindestens 80 Gew.-%, bezogen auf das Gesamtgewicht der trägerfreien Formkörper.

Die VI. Nebengruppe des Periodensytems enthält die Elemente Chrom, Molybdän und Wolfram. Die erfindungsgemäß zu verwendenden trägerfreien Formkörper enthalten eines oder mehrerer dieser Metalle in Mengen von 0 - 15 Gew.-%. Für den Fall ihres Vorliegens enthalten die Metallpulver mindestens 0,1, vorzugsweise mindestens 0,3, insbesondere mindestens 0,5 Gew.-%, bezogen auf trägerfreie Formkörper; sie enthalten eines oder mehrere dieser Metalle in Mengen von höchstens 15, vorzugsweise höchstens 10 und insbesondere höchstens 5 Gew.-%, bezogen auf trägerfreie Formkörper.

Die erfindungsgemäß zu verwendenen trägerfreien Formkörper können darüber hinaus - jeweils bezogen auf trägerfreie Formkörper - bis zu 25, vorzugsweise bis zu 15 Gew.-% andere Elemente enthalten; Beispiele solcher Elemente, die nicht katalytisch wirken, umfassen Aluminium, Silicium, Titan und Kohlenstoff. Nach einer bevorzugten Ausführungsform enthalten die trägerfreien Formkörper außer den Metallen der VIII. und VI. Nebengruppe nicht mehr als 10 Gew.-% Aluminium und nicht mehr als 5 Gew.-% anderen Elemente.

Für den Hydrierprozeß wird auf einen Druck von 50 - 400 bar, bevorzugt 100 bis 300 bar, vorkomprimierter reiner Wasserstoff eingesetzt, wobei man mit einer 20-bis 60-fachen, bevorzugt 20- bis 40-fachen molaren Wasserstoffmenge, bezogen auf die stöchiometrische Menge, arbeitet.

Die Hydrierung erfolgt kontinuierlich im Festbettverfahren an den als Hydrierungskatalysatoren dienenden trägerfreien Formkörpern der beschriebenen Art, indem man die zu hydrierenden flüssigen Maleinsäure-dialkylester entweder im Gleichstrom mit dem zuvor zugemischten Wasserstoff von unten nach oben aufsteigend über die in den Hydrierreaktor gefüllten Formkörper strömen läßt oder auch dem von oben einströmenden Wasserstoff von unten kommend entgegenführt (Gegenstromverfahren).

Das erfindungsgemäße Verfahren kann natürlich auch in Lösungsmitteln durchgeführt werden. Geeignete unter den Reaktionsbedingungen inerte Lösungsmittel sind beispielsweise Di-n-propyl-ether, Di-iso-propyl-ether, Di-n-butyl-ether, Tetrahydrofuran, Dioxan, γ-Butyrolacton. Es kann auch in Gegenwart des zu bildenden Bernsteinsäure-dialkylesters gearbeitet werden.

Der Hydrierprozeß wird bei Temperaturen von 30 bis 160°C, vorzugsweise 40 bis 100°C, durchgeführt. Niedrigere Temperaturen bedingen höhere Verweilzeiten oder den Verzicht auf einen quantitativen Umsatz. Höhere Temperaturen führen zur Bildung von Butandiol-1,4 und Esteralkohol als Nebenprodukte.

Die stündliche Katalysatorbelastung kann 200 bis 500 g Maleinsäure-dialkylester/l Katalysator betragen.

Der Hydrierreaktor kann entweder ein einzelnes Hochdruckrohr aus Stahl oder einer Stahllegierung sein, das mit den trägerfreien Formkörpern ganz oder teilweise gefüllt wird, wobei auch die Anwendung auf Horden (Drahtkörbe o.ä.) nützlich sein kann, oder aber ein ummanteltes Hochdruckrohrbündel, dessen Einzelrohre mit Formkörpern ganz oder teilweise gefüllt werden.

Die Herstellung der trägerfreien Formkörper kann nach gebräuchlichen Methoden durch Verpressen der Metallpulver auf Tablettier- und Pelletiermaschinen unter hohem Druck erfolgen, wobei zur Verbesserung des Haftvermögens der Metallpartikel auch Graphit in Mengen von 0,5 - 1,5 Gew.-%, bezogen auf das Gesamtgewicht der den Katalysator bildenden Bestandteile, oder Klebestoffe in kleinen Mengen zum Einsatz kommen können. Die Herstellung der trägerfreien Formkörper erfolgt vorzugsweise in einer sauerstofffreien Atmosphäre, um Oberflächenoxidationen zu vermeiden. Am wirksamsten und für die Reaktionsführung am günstigsten sind tablettierte und pelletierte Formkörper mit Durchmessern von 3 bis 7 mm. Von erheblicher Bedeutung ist die Druckfestigkeit der Formkörper, die erfindungsgemäß bei Werten von 20 bis 250 N, bevorzugt 110 bis 220 N, liegt. Niedrigere Druckfestigkeiten führen zu Formkörperzerfall bzw. erosivem Abrieb, was eine metallische Kontaminierung des Reaktionsproduktes bewirken würde. Höhere Werte bedingen einen unangemessenen Aufwand beim Verpressen, ohne daß weitere Vorteile erzielt werden. Von erheblicher Bedeutung ist weiterhin die innere Oberfläche der Formkörper, die erfindungsgemäß bei Werten von 10 bis 90 m²/g liegt und ausschlaggebend für einen möglichst quantitativen Umsatz der Einsatzstoffe ist. Die Formkörper haben makroskopisch eine glatte Oberfläche.

Unter den geschilderten Reaktionsbedingungen sind auf diese Weise ganz unerwartet hohe Katalysatorstandzeiten von 15 000 Stunden und mehr zu erzielen, was zu Katalysatorverbräuchen < 0,1 Gew.-%, bezogen auf hergestelltes Reaktionsprodukt, führt; so niedrige Katalysatorverbräuche konnten bisher bei der Hydrierung von Maleinsäureestern noch nicht erreicht werden.

Das den Hydrierreaktor verlassende Reaktionsgemisch wird entspannt, wobei man den überschüssigen Wasserstoff abfangen und nach erfolgtem Komprimieren und Ergänzung von verbrauchtem Wasserstoff erneut zum Einsatz bringen kann. Bei einem 99,9 bis 100 %igen Umsatz besteht das Reaktionsgemisch zu mindestens 99 Gew.-% aus Bernsteinsäure-dialkylestern.

Die erfindungsgemäß einzusetzenden sauerstofffreien und trägerfreien Festbettkatalysatoren neigen im Gegensatz zu trägerhaltigen Katalysatoren nicht zum "Ausbluten", d.h. nicht zum Übergang von Katalysatorbestandteilen in ionischer oder kolloidaler Form in die Lösungsphase des Substrats, so daß das Substrat nicht durch Schwermetalle kontaminiert wird, die normalerweise ebenfalls nur mühsam, beispielsweise mit Hilfe von Ionenaustauschern, aus dem Substrat erntfernt werden können. Die einzusetzenden Katalysatormetalle können, etwa nach längerem Gebrauch des Katalysators leicht aufgearbeitet und wiederverwendet werden, da die Schwermetalle nicht umständlich von einem Trägermaterial getrennt werden müssen. Bei polyfunktionellen Verbindungen, beispielsweise bei nur teilweise veresterten mehrwertigen Alkoholen, war weiterhin die Neigung zu befürchten, daß mit Schwermetallionen komplexe Chelatverbindungen der Fettseifen gebildet werden, die nur schwierig aus den Estern oder Estergemischen entfernt werden können; diese Befürchtung tritt mit den erfindungsgemäß einzusetzenden Katalysatoren nicht ein.

Die erzeugten Bernsteinsäure-dialkylester werden nach der destillativen Entfernung eines geringfügigen Leichtsiedervorlaufs und gegebenenfalls Nachlaufs in einer Reinheit von ≥99,9 Gew.-% erhalten und sind in dieser Qualität für alle weiterverarbeitenden Prozesse einsetzbar.

### Beispiele

### Beispiel 1

Ein senkrecht stehendes, wärmeisoliertes Hochdruckrohr aus nichtrostendem Stahl von 45 mm Innendurchmesser und 1 m Länge wurde mit 1,4 l eines durch Tablettierung von Ni-Pulver hergestellten Hydrierungskatalysators gefüllt, der bei einer Zylinderhöhe von 5 mm und einem Durchmesser von 5 mm eine Druckfestigkeit von 147 N auf die Zylindermantelfläche und eine innere Oberfläche von 33 m²/g aufwies. Durch dieses Rohr wurden stündlich 480 g Maleinsäure-dimethylester (Reinheit ≥99,5 Gew.-%) gemeinsam mit der 20-fachen molaren Menge von unter einem Druck von 300 bar stehendem, hochreinem Wasserstoff von unten nach oben aufsteigend gepumpt.

Maleinsäure-dimethylester und Wasserstoff wurden vorab gemeinsam durch einen Wärmeaustauscher geführt und so erwärmt, daß sie in das Hochdruckrohr mit einer Temperatur von 40°C eintraten. Das das Hochdruckrohr verlassende Gemisch aus flüssigem Reaktionsprodukt und überschüssigem Wasserstoff wurde in einen Abscheider geführt, von wo der Wasserstoff nach Ersatz der verbrauchten Menge wieder zusammen mit neuem Maleinsäure-dimethylester in den Vorwärmer und von dort erneut in das Hochdruckrohr gepumpt wurde.

Die farblose und klare Flüssigkeit des Reaktionsproduktes wurde nach Entspannung auf Normaldruck und Abkühlung gaschromatographisch untersucht. Sie enthielt an alkoholischen Nebenbestandteilen 0,2 Gew.-% Methanol und 0,1 Gew.-% Butandiol-1,4. Der Maleinsäure-dimethylestergehalt des organischen Reaktionsproduktes betrug <0,1 Gew.-%, so daß der Bernsteinsäure-dimethylester-gehalt bei >99,6 Gew.-% lag. Der erzeugte Bernsteinsäure-dimethylester wurde nach der destillativen Entfernung der Nebenbestandteile in einer Reinheit von 99,9 Gew.-% erhalten und besaß einen Siedepunkt von 196°C.

### Beispiel 2

In einem Hochdruckrohr wie in Beispiel 1 wurde bei einer Temperatur von 45°C und einem Wasserstoffdruck von 200 bar der Wasserstoff im umgekehrten Reaktionsfluß wie in Beispiel 1 dem aufsteigenden Maleinsäure-dimethylester entgegengeführt, wobei stündlich eine gleich große Menge wie in Beispiel 1 hydriert wurde. Der Katalysator war durch Tablettierung einer pulverisierten Ni/Fe-Legierung hergestellt worden. Die Legierung enthielt einen Eisenanteil in Nickel von 15 Gew.-%. Die Tabletten hatten bei einer Zylinderhöhe von 5 mm und einem Durchmesser von 5 mm eine Druckfestigkeit von 137 N auf die Zylindermantelfläche und eine innere Oberfläche von 74 m²/g.

Nach einer Laufzeit von 3400 Stunden lag der Umsatz des eingesetzten Maleinsäure-dimethylesters bei 99,95 Gew.-%. Der Gehalt an Methanol im Reaktionsprodukt lag bei 0,20 Gew.-% und der Butandiol-1,4-Gehalt bei 0,15 Gew.-%, so daß der Bernsteinsäure-dimethylestergehalt des Reaktionsproduktes bei 99,60 Gew.-% lag. Nach der destillativen Entfernung der Verunreinigungen wurde der erzeugte Bernsteinsäure-dimethylester in einer Reinheit von 99,9 Gew.-% erhalten.

### Beispiel 3

Ein senkrecht stehendes, wärmeisoliertes Hochdruckrohr aus nichtrostendem Stahl von 45 mm Innendurchmesser und 1 m Länge wurde mit 1,4 l eines durch Tablettierung von Pulver einer Ni/Mo-Legierung mit einem Mo-Gehalt von 1,75 % hergestellten Hydrierungskatalysators gefüllt, der bei einer Zylinderhöhe von 5 mm und einem Durchmesser von 5 mm eine Druckfestgkeit von 191 N und eine innere Oberfläche von 58 m²/g aufwies. Durch dieses Rohr wurden stündlich 590 g Maleinsäure-diethylester gemeinsam mit der 30-fachen molaren Menge von unter einem Druck von 300 bar stehendem hochreinen Wasserstoff von unten nach oben aufsteigend gepumpt.

Maleinsäure-diethylester und Wasserstoff wurden vor Eintritt in das Hochdruckrohr auf eine Temperatur von 55°C gebracht.

Nach einer Laufzeit von 1600 Stunden lag der Umsatz des eingesetzten Maleinsäure-diethylesters bei 100 Gew.-%. Der Gehalt an Ethanol im Reaktionsprodukt lag bei 0,3 Gew.-% und der Gehalt an Butandiol-1,4 bei 0,1 Gew.-%, so daß der Bernsteinsäure-diethylestergehalt bei 99,6 Gew.-% lag. Nach der destillativen Entfernung der Verunreinigungen wurde der gewonnene Bernsteinsäure-diethylester in einer Reinheit von 99,9 Gew.-% erhalten. Er besaß einen Siedepunkt von 217°C.

### Beispiel 4

In einem Hochdruckrohr wie in Beispiel 1 wurde bei einer Temperatur von 45°C und einem Wasserstoffdruck von 200 bar stündlich eine gleich große Menge Maleinsäure-diethylester hydriert. Der Katalysator wurde durch Tablettierung einer pulverisierten Ni/Fe/Mo-Legierung hergestellt. Die Legierung enthielt einen Fe-Anteil in Ni von 15 % sowie einen Mo-Gehalt von 1,4 %. Die Tabletten hatten bei einer Zylinderhöhe von 3 mm und einem Durchmesser von 3 mm eine Druckfestigkeit von 162 N und eine innere Oberfläche von 68 m²/g.

Nach einer Laufzeit von 1600 Stunden lag der Umsatz des eingesetzten Maleinsäure-diethylesters bei 99,9 Gew.-%. Der Gehalt an Ethanol im Reaktionsprodukt betrug 0,2 Gew.-% und der Gehalt an Butandiol-1,4 0,1 Gew.-%, so daß der Bernsteinsäure-diethylestergehalt des Reaktionsproduktes bei 99,6 Gew.-% lag.

### Beispiel 5

In einem Hochdruckrohr wie in Beispiel 1, aber aus Hochdruckstahl N 9, wurde bei einer Temperatur von 50°C und einem Wasserstoffdruck von 300 bar stündlich eine gleich große Menge Maleinsäure-di-n-butylester hydriert. Der Katalysator wurde durch Tablettierung von Pulver einer Ni/Mo/Al-Legierung mit einem Mo-Gehalt von 1,02 Gew.-% und einem Al-Gehalt von 5,1 Gew.-% hergestellt. Die Tabletten hatten bei einer Zylinderhöhe von 5 mm und einem Durchmesser von 5 mm eine Druckfestigkeit von 210 N und eine innere Oberfläche von 71 m²/g.

Nach einer Laufzeit von 2400 Stunden lag der Gehalt an Bernstein-di-n-butylester im Reaktionsprodukt bei 99,6 Gew.-% Nach der destillativen Entfernung der Verunreinigungen wurde der erzeugte Bernsteinsäure-di-n-butylester in einer Reinheit von 99,9 Gew.-% erhalten (Kp₄: 108°C).

### Beispiel 6

In einem Hochdruckrohr wie in Beispiel 1 wurde bei einer Temperatur von 45°C und einem Wasserstoffdruck von 300 bar stündlich eine Menge von 360 g Maleinsäure-dimethylester, hydriert. Der Katalysator wurde durch Tablettierung einer pulverisierten Ni/Al-Legierung mit einem Al-Gehalt von 6,1 Gew.-% hergestellt. Die Tabletten hatten bei einer Zylinderhöhe von 3 mm und einem Durchmesser von 3 mm eine Druckfestigkeit von 156 N auf die Zylindermantelfläche und eine innere Oberfläche von 69 m²/g.

Nach einer Laufzeit von 1140 Stunden lag der Umsatz des eingesetzten Maleinsäure-dimethylesters bei 99,9 Gew.-%. Nach der Destillation des rohen Bernsteinsäure-dimethylesters hatte dieser eine Reinheit von 99,9 Gew.-% und einen Siedepunkt von 196°C.

### Beispiel 7

In einem Hochdruckrohr wie in Beispiel 1 wurde bei einer Temperatur von 55°C und einem Wasserstoffdruck von 200 bar stündlich eine Menge von 420 g Maleinsäure-di-n-propylester hydriert. Der Katalysator wurde durch Tablettierung einer pulverisierten Ni/Al/Si-Legierung mit einem Al-Gehalt von 5,4 Gew.-% und einem Si-Gehalt von 0,2 Gew.-% gewonnen. Die Tabletten hatten bei einer Zylinderhöhe von 3 mm und einem Durchmesser von 3 mm eine Druckfestigkeit von 148 N und eine innere Oberfläche von 61 m²/g.

Nach einer Laufzeit von 1900 Stunden lag der Umsatz des eingesetzten Maleinsäure-di-n-propylesters bei >99,0 Gew.-%. Der Gehalt an n-Propanol im Reaktionsprodukt betrug 0,3 Gew.-% und der Gehalt an Butandiol-1,4 0,18 Gew.-%, so daß der Bernsteinsäure-di-n-propylestergehalt bei ≥99,42 Gew.-% lag. Nach der Destillation des rohen Bernsteinsäure-di-n-propylesters hatte dieser einen Siedepunkt von 248°C.

## Patentansprüche

1. Verfahren zur kontinuierlichen Herstellung von Bernsteinsäure-dialkylestern durch katalytische Hydrierung von Maleinsäure-dialkylestern, dadurch gekennzeichnet, daß die Hydrierung in der flüssigen Phase bei einem H₂-Druck von 50 bis 400 bar, einer 20 bis 60-fachen molaren H₂-Menge, bezogen auf die stöchiometrische Menge, und bei einer Temperatur von 30 bis 160°C an im Festbett angeordneten sauerstofffreien und trägerfreien Katalysatoren durchgeführt wird, die als verpreßte aus Metallpulvern hergestellte Formkörper vorliegen, die eine Druckfestigkeit von 20 bis 250 N und eine innere Oberfläche von 10 bis 80 m²/g aufweisen und bei denen die Metallpulver 60 bis 100 Gew.-% eines oder mehrerer Eisenmetalle, 0 bis 15 Gew.-% eines oder mehrerer Metalle der VI. Nebengruppe und 0 bis 25 Gew.-% eines oder mehrerer hydrierinerter Elemente aus der Gruppe von Aluminium, Silicium, Titan und Kohlenstoff, alles bezogen auf das Gesamtgewicht des Metallpulvers, enthalten.

2. Verfahren nach Anspruch 1, dadurch gekennzeichnet, daß die Metallpulver 70 bis 100 Gew.-%, bevorzugt 80 bis 100 Gew.-%, eines oder mehrerer Eisenmetalle enthalten.

3. Verfahren nach Anspruch 1, dadurch gekennzeichnet, daß die Metallpulver beim Vorliegen von Metallen der VI. Nebengruppe einen Gehalt von 0,1 bis 15 Gew.-%, bevorzugt von 0,3 bis 10 Gew.-%, besonders bevorzugt von 0.5 bis 5 Gew.-%, daran enthalten.

4. Verfahren nach Anspruch 1, dadurch gekennzeichnet, daß die Metallpulver beim Vorliegen von hydrierinerten Elementen einen Gehalt von 0 bis 10 Gew.-% an Aluminium und von 0 bis 5 Gew.-% je Element Si, Ti und C enthalten.

5. Verfahren nach Anspruch 4, dadurch gekennzeichnet, daß der Gesamtgehalt der hydrierinerten Elemente 0 bis 15 Gew.-%, bevorzugt 0 bis 10 Gew.-%, beträgt.

6. Verfahren nach Anspruch 1, dadurch gekennzeichnet, daß es sich bei den Formkörpern um solche mit einer Druckfestigkeit von 110 bis 220 N handelt.

7. Verfahren nach Anspruch 1, dadurch gekennzeichnet, daß die Formkörper zylinder- oder kugelförmig sind und Durchmesser von 3 bis 7 mm besitzen.

8. Verfahren nach Anspruch 1, dadurch gekennzeichnet, daß die Hydrierung bei einem H₂-Druck von 100 bis 300 bar durchgeführt wird.

9. Verfahren nach Anspruch 1, dadurch gekennzeichnet, daß bei einer 20 bis 40-fachen molaren H₂-Menge gearbeitet wird.

10. Verfahren nach Anspruch 1, dadurch gekennzeichnet, daß der zu hydrierende ungesättigte Fettsäureester den Hydrierreaktor von unten nach oben aufsteigend passiert, während der für die Hydrierung benötigte Wasserstoff entweder gemeinsam mit dem ungesättigten Ester in den Reaktor gepumpt wird oder diesem, von oben nach unten strömend, entgegengeführt wird.

## Claims

1. Process for the continuous preparation of succinic acid dialkyl esters by catalytic hydrogenation of maleic acid dialkyl esters, characterized in that the hydrogenation is carried out in the liquid phase under an H₂ pressure of 50 to 400 bar, with 20 to 60 times the molar amount of H₂, based on the stoichiometric amount, and at a temperature of 30 to 160°C over oxygen-free and support-free catalysts which are arranged in a fixed bed, are present in the form of compressed shaped bodies produced from metal powders and having a compressive strength of 20 to 250 N and an internal surface area of 10 to 80 m²/g, and in which the metal powders comprise 60 to 100% by weight of one or more iron metals, 0 to 15% by weight of one or more metals of sub-group VI and 0 to 25% by weight of one or more hydrogenation-inert elements from the group consisting of aluminium, silicon, titanium and carbon, all based on the total weight of the metal powder.

2. Process according to Claim 1, characterized in that the metal powders comprise 70 to 100% by weight, preferably 80 to 100% by weight, of one or more iron metals.

3. Process according to Claim 1, characterized in that, if metals of sub-group VI are present, the metal powders comprise a content of 0.1 to 15% by weight, preferably 0.3 to 10% by weight particularly preferably 0.5 to 5% by weight, thereof.

4. Process according to Claim 1, characterized in that, if hydrogenation-inert elements are present, the metal powders comprise a content of 0 to 10% by weight of aluminium and of 0 to 5% by weight of each of the elements Si, Ti and C.

5. Process according to Claim 4, characterized in that the total content of the hydrogenation-inert elements is 0 to 15% by weight, preferably 0 to 10% by weight.

6. Process according to Claim 1, characterized in that the shaped bodies are those having a compressive strength of 110 to 220 N.

7. Process according to Claim 1, characterized in that the shaped bodies are cylindrical or spherical and have diameters of 3 to 7 mm.

8. Process according to Claim 1, characterized in that the hydrogenation is carried out under an H₂ pressure of 100 to 300 bar.

9. Process according to Claim 1, characterized in that it is carried out with 20 to 40 times the molar amount of H₂.

10. Process according to Claim 1, characterized in that the unsaturated fatty acid ester to be hydrogenated is passed through the hydrogenation reactor from the bottom upwards, while the hydrogen required for the hydrogenation is either pumped into the reactor together with the unsaturated ester or passed against this from the top flowing downwards, and an internal surface area of 10 to 80 m²/g.

## Revendications

1. Procédé pour la préparation en continu d'esters dialkyliques de l'acide succinique par hydrogénation catalytique d'esters dialkyliques de l'acide maléique, caractérisé en ce qu'on effectue l'hydrogénation en phase liquide sous une pression de H₂ de 50 à 400 bar, avec une quantité de H₂ de 20 à 60 fois molaire rapportée à la quantité stoechiométrique et à une température de 30 à 160°C sur des catalyseurs exempts d'oxygène et sans support, disposés en lit fixe, qui se présentent sous la forme de corps moulés comprimés préparés à partir de poudres métalliques, qui présentent une résistance à la pression de 20 à 250 N et une surface interne de 10 à 80 m²/g et dans lesquels les poudres métalliques contiennent, à concurrence de 60 à 100% en poids, un ou plusieurs métaux ferreux, à concurrence de 0 à 15% en poids, un ou plusieurs métaux de la VIème famille secondaire et à concurrence de 0 à 25% en poids, un ou plusieurs éléments hydrogénés choisis parmi le groupe comprenant l'aluminium, le silicium, le titane et le carbone, respectivement rapportés au poids total de la poudre métallique.

2. Procédé selon la revendication 1, caractérisé en ce que les poudres métalliques contiennent un ou plusieurs métaux ferreux à concurrence de 70 à 100% en poids, de préférence de 80 à 100% en poids.

3. Procédé selon la revendication 1, caractérisé en ce que les poudres métalliques, en présence de métaux de la VIème famille secondaire, possèdent ces derniers en une teneur de 0,1 à 15% en poids, de préférence de 0,3 à 10% en poids, de manière particulièrement préférée de 0,5 à 5% en poids.

4. Procédé selon la revendication 1, caractérisé en ce que les poudres métalliques, en présence d'éléments hydrogénés, possèdent une teneur en aluminium de 0 à 10% en poids et chacun des éléments Si, Ti et C de 0 à 5% en poids.

5. Procédé selon la revendication 4, caractérisé en ce que la teneur totale des éléments hydrogénés s'élève de 0 à 15% en poids, de préférence de 0 à 10% en poids.

6. Procédé selon la revendication 1, caractérisé en ce qu'il s'agit, en ce qui concerne les corps moulés, de ceux possédant une résistance à la pression de 110 à 220 N.

7. Procédé selon la revendication 1, caractérisé en ce que les corps moulés sont de forme cylindrique ou sphérique et possèdent un diamètre de 3 à 7 mm.

8. Procédé selon la revendication 1, caractérisé en ce qu'on effectue l'hydrogénation sous une pression de H₂ de 100 à 300 bar.

9. Procédé selon la revendication 1, caractérisé en ce qu'on travaille avec une quantité de H₂ de 20 à 40 fois molaire.

10. Procédé selon la revendication 1, caractérisé en ce qu'on fait passer les esters d'acides gras insaturés à hydrogéner à travers le réacteur d'hydrogénation en les faisant monter de bas en haut tandis que l'hydrogène requis pour l'hydrogénation, soit est pompé dans le réacteur de manière conjointe avec l'ester insaturé, soit est guidé de haut en bas à contre-courant par rapport à l'ester insaturé sous forme d'un courant descendant.
